# EUROPEAN PATENT APPLICATION

(11) **EP 2 737 883 A2**
(43) Date of publication of application: **04.06.2014**
(21) Application number: 13152598.2
(22) Date of filing: 24.01.2013
(51) Int. Cl.: A61F 7/00, H05B 3/34, A61F 7/08

(54) **Heating system for patient thermal management**

(30) Priority: 04.01.2012 US 201213374625
(71) Applicant: ThermoGear, Inc., Lake Oswego, OR 97035 (US)
(72) Inventor: Mills, Gary N., Gladstone, oregon 97027 (US)
(74) Representative: Freeman, Jacqueline Carol

(57) **Abstract**

An electrifiable heating blanket for patients, particularly including small animals, undergoing surgery. Heating of the blanket is varied in accordance with patient body temperature changes. Control of the heating is provided by a control unit made accessible to a care giver (surgeon or assistant), and blanket temperature is varied by increasing/decreasing electrical current flow to the blanket via the control unit. A primary conductor path is provided from the electrical source to a base control unit and from the base control unit to the blanket covering the patient. The invention provides a secondary conductor path from a position along the primary conductor path and independently extended to a position of convenience to the surgeon or assistant. An electronic break out feature is provided at the point of diversion enabling electronics push button control from the hand control.

## Description

### FIELD OF INVENTION

This invention relates to heating systems for heating garments, covers, pads and the like (hereafter inclusively referred to as "cover" or "covering"), used to heat persons and /or animals e.g., having medical needs (collectively referred to herein as "patients") where personalized heating is desirable.

### BACKGROUND OF THE INVENTION

Heating systems of the type herein referred to are disclosed in prior, commonly owned patents. Included are U.S. 5,986,243 directed to heating persons attending outdoor events; U.S. 7,959,658 directed to heating persons injured at remote locations, and U.S. 7,319,207 directed to hospitalized patients being transported between different in-hospital locations where continued warming is desired. The disclosures of the above patents are herein included by reference.

These systems all have common components including a blanket, pad or other type covering that is provided with electrically induced heating elements, a source of DC current, a conductor transmitting DC current to the covering, and a manually adjustable control that controls the temperature of the covering.

### SUMMARY OF THE INVENTION

The present invention relates to the control feature for manually controlling the heat temperature provided to the covering. As will be appreciated by those skilled in the art, controlling the temperature involves increasing and decreasing current flow to the covering. The control feature therefore controls the extent of electrical current flow.

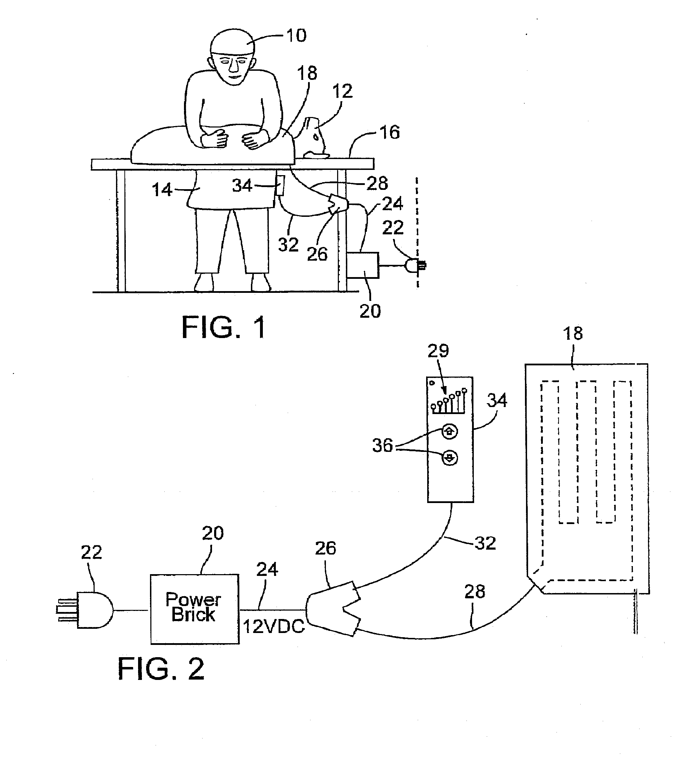

The above feature (DC current control) has been provided as a common component of the personal heating systems of prior art and patents applicable thereto. Whereas supply of current required a current conductor extending between a current source and a blanket, the current control component was provided along the length of the current conductor (e.g. control unit 20 of the 207 patent). As can be noted from fig 1 of the '207 patent, a heat control unit 20, receiving DC current from conductor 34, includes a control dial 26 manipulated by an operator, the controlled heat being conveyed to a covering 12 by conductor 18.

The present invention resulted from an appreciation that manipulation of the temperature was important during e.g., a surgical process, but also often an undesired and inconvenient interruption e.g., to a surgeon and/or attendant having a primary objective to perform an on-going medical procedure.

The embodiment of a surgical heating cover herein disclosed, is controlled by a separate component e.g., a hand control unit that is electrically or electronically connected to the primary controller component, but physically located separately e.g., on or near the care giver. The hand control enables control of the covering's temperature and is connected by a separate conductor removed from the path of the conductor that is extended between the electrical source and the covering.

The invention and benefits thereof will be more fully appreciated upon reference to the following detailed description and drawings of a preferred embodiment of the invention.

### DESCRIPTION OF THE DRAWINGS

Fig 1 is an illustration of a medical procedure incorporating an embodiment of the present invention;
Fig 2 is a schematic illustration illustrating an example of the warming covering and heat system therefore;
Fig 3 illustrates, in schematic form, the invention as embodied in figs 1 and 2;
Fig 4 illustrates a dual component arrangement of the invention that replaces the conventional battery/control component of the prior art heated coverings;
Fig. 5 illustrates, in schematic form, an alternative embodiment of the invention; and
Fig. 6 is an illustration of an electrical system for achieving the desired control as explained in the description that follows.

### DESCRIPTION OF A PREFERRED EMB0DIMENT

With reference to fig 1 of the drawings, illustrated is a veterinarian 10 as when performing a medical procedure on an animal patient 12. The vet 10 is wearing a surgical gown 14 and the patient (animal) 12 is lying on table 16. A warming covering 18 is provided for the patient.

As known to the art (see the patents referred to above) the blanket is provided with DC current compatible warming elements. It will be here noted that DC current is preferred for warming of a blanket, pad, garment or whatever covering is applied to the patient. In the U.S. the most available external electrical power is AC current. Whereas AC power is likely available and DC may not be available, the system may preferably include the provision of a supplemental DC power source. Sources known are power suppliers that convert AC to DC current but also very common and readily available are batteries. Either or both can be incorporated into the systems herein disclosed.

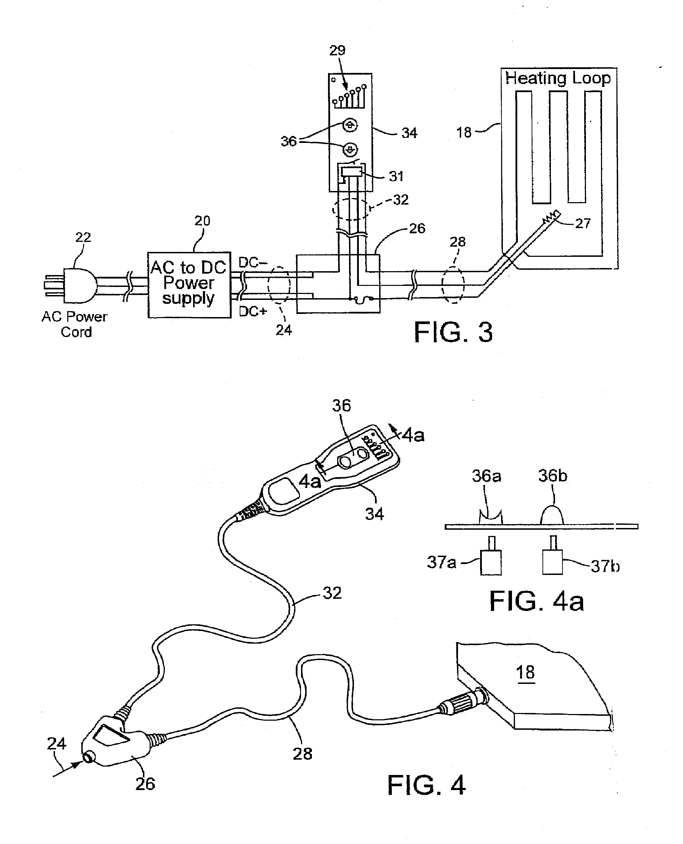

Reference is now made to figs 2 and 3. In fig 2 the system or concept that exemplifies the invention is illustrated schematically. In this illustration the DC power source is a power brick 20 that converts AC current from AC plug-in 22 (connected to an AC outlet) to DC current that is conducted via conductor 24 to a controller component 26.

As shown in figs 2 and 3, there are two conductors or conductor segments 28 and 32 extending from the controller component 26. Conductor 28 extends to the covering 18 (e.g., mat, blanket, gown) and conductor 32 extends to a hand control 34. The electronics are best illustrated in fig 3 and persons' skilled in the art will readily discern the wiring diagram of fig 3 for duplicating the arrangement desired i.e. the separation of the hand control component 34 from the control component 26 (sometimes referred to as a breakout box). It will also be apparent that conductors 24 and 28 includes DC - and DC+ wiring as illustrated, with the DC- phase being diverted at the breakout box 26, through conductor 32, to the hand control 34, and then back to the controller component 26. As also illustrated and apparent from fig 3, the conductor 28 conducts DC - current from hand control 34 and controller 26, and DC + current directly from the component 26 to the covering 18. The electronics of the system will be readily discerned by persons skilled in the art with reference to the drawings.

With the breakout of the DC current at the controller component 26 and diverting the DC current first to the hand control 34 and then back to the controller component 26, such enables manipulation (adjustment) of the heat temperature remotely from the location of the controller component 34. As will be apparent to persons skilled in the art, a thermistor 27 embedded in the covering 18 monitors the temperature of the blanket. Temperature probes may be attached to the patient to enable the caregiver to determine whether increases or decreases of the covering temperature are desired. The up down change to the temperature is manipulated by pressing up - down buttons 36.

Reference is now directed to figs 1, 2 and 3. From the above description, it will be appreciated that a care giver 10 can provide the desired warming of the patient 12 via DC conductor 32 connected to the controller 26 and then conductor 28 connected to warming covering 18. Should adjustment of the temperature be desired, the care giver 10 needs merely to reach down to the hand control 34 (see fig. 1) and vary the DC current as enabled by the manipulation of switch 36, illustrated in figures 2, 3, and 4.

The switch 36 activation is more clearly illustrated in figs 4 and 4a. Pressing the button 36a of the switch 36 produces engagement of sensor 37a to lower the temperature set point, and pressing the button 36b of the switch 36 produces the engagement of sensor 37b to raise the temperature set point. Not shown is the means for enabling the caregiver 10 to secure the hand control where desired e.g., to the table 16 or to his clothing 14 if desired e.g., via a clip, Velcro etc., provided on the back side of the hand control 34. In any event the provision of the hand control 34 being physically independent of the controller 26 (but to a limited degree), and via the clip or other securement device, the hand control can be easily and conveniently secured to table 16, clothing 14, or otherwise as the care giver finds most convenient.

Whereas the separation and positioning of the hand control being independent of the conductor path between the power source and the covering is unique per se, the manner by which that control is achieved has additional benefit and uniqueness. That is, the control as designed, reduces the number of wires required and thereby achieves a wire wrap (e.g., conductor segments 24,28, 32) that has greater flexibility with easier movement and location of the tied components i.e., the covering, hand control and break out wye.

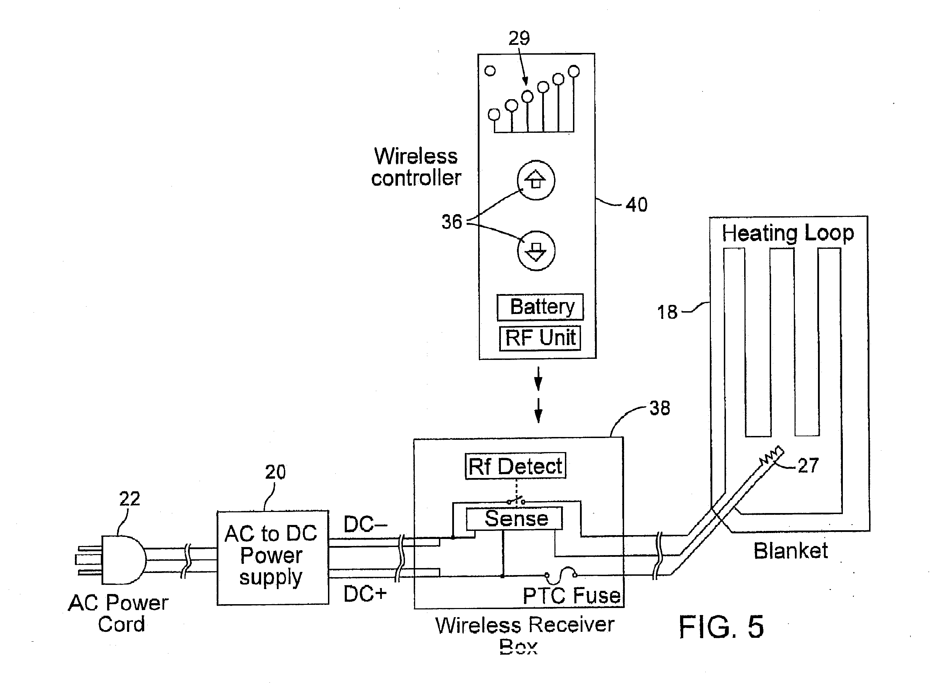

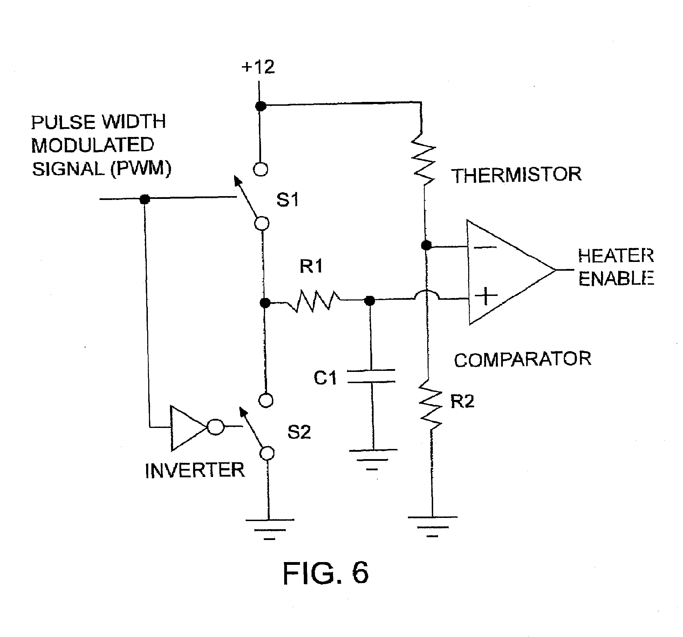

With reference to fig 3, (but refer also to fig. 6), it will be noted that the thermistor 27 is in series with a resistor and is tied between plus 12 volts and ground. (Note the pathway of the conductor wires extending from the thermistor 27, through wire 28 and into the wye breakout 26, and then to the hand control 34.) This arrangement reduces the number of wires required but in doing so creates an issue of stability i.e., the e.g., 12 volts of the DC source is not stable due to the varying load that is encountered. The stability issue is resolved using a pulse width modulator (pwm) 31. (Also see item titled "Sense" in fig 5.) As an example, the modulator may be set e.g., to 55%. In this way it does not matter what the 12 volts actually is, as the system merely tracks the desired ratio. This ratio is then increased or decreased by e.g., the care giver via manipulation of control 36.

The thermistor resistance (TI) changes with temperature. The higher the temperature the lower its resistance. Therefore the voltage on the minus input or the comparitor goes up as the temperature increases. There is a known relationship between this voltage and temperature. Note that this voltage is proportional to the voltage of the +12 volt line. It will be appreciated that a voltage can be created that represents the desired temperature by pulse width modulating S 1 and S2 (See fig 6). The pulse signal on the node between S1 and S2 is filtered by R1 and C 1 to create a DC signal representing the desired temperature. The percentage on time of the PWM signal is set by the user control which determines the desired temperature. The desired temperature and the actual temperature feed a comparater to determine if the heater should be enabled.. Because S1 and S2 operate between +12 and ground and the thermistor and resistor are tied between+12 and ground the temperature is independent of the voltage on the + 12 line, permitting proper operation as the actual voltage varies from the nominal +12 volts.

Further note from fig 4a that the switch 36b is protruded (convex shaped) and 36a is depressed (concave shaped). Thus the care giver need merely (even through surgical gloves) to feel the different shapes and thereby readily control the up or down positions and make the desirable adjustments, with minimal interruptions to his primary activity (operation).

An example of a likely variation is illustrated in fig 5. The electronics are best comprehended by a person skilled in the art having reference to the drawing of fig 5. Similar to the illustration of fig 3, a power source 20 provides DC current to a wireless receiver box 38 (as differentiated form the wye breakout box 26). The electronics are different in that the box 38 receives electronic signals remotely, wireless, from the hand control 40. The signal for controlling the temperature of covering 18 is achieved remotely as will be apparent again to persons skilled in the art. Essentially the manner of control is similar and further explanation is deemed unnecessary.

As previously indicated, the invention as disclosed is applicable to electrifiable heated coverings (pads, blankets, body suits, and the like) e.g., as disclosed in the above cited prior patents ('243; '658; '207). Of further note is the availability of a covering produced from electrifiable fabric which is disclosed in U.S. Patent Application Publication No.; U.S. 2007/0049997 A1. Publication Date; March 1, 2007. (This disclosure is the subject of a commonly owned pending patent application.) The disclosure in its entirety is incorporated herein by reference.

It is further noted that although the invention has beneficial application to heating persons and animals in general, there is considered to be perhaps a greater need for the treatment of animals. Veterinarians are considered less likely to have assistants in attendance and are more likely to be the person having to attend to the changing of temperature settings of the animal being treated. Nevertheless, the invention is considered to have general application to all categories of patients being treated (surgery) by a caregiver.

It is to be understood that the above disclosure of the preferred embodiments are but examples of the invention embodied in e.g., a medical procedure. Numerous variations and improvements may be made without departing from the essence of the invention. Accordingly the scope of the invention is to be determined by the claims appended hereto.

## Claims

1. A patient heating system comprising:
a covering responsive to electrical current input to produce warming of a patient;
a multi-segment electrical current conductor including a first conductor segment for receiving electrical current from an electrical power source;
a dual component controller, a first component of the controller receiving current from said first conductor segment and transmitting current from said first component through a second conductor segment to the covering;
a second component of the dual component controller comprising a hand control separate from and electrically connected to said first component by a third conductor segment, and said hand control including manually controllable adjustment of the temperature ;
said second component as electrically connected to the first component permitting limited independent positioning of the band control as related to the first component and the covering, whereby a caregiver can initiate variable heating of the covering from a desired care giver position that is remote from the first component

2. A patient heating system as defined in claim 1 wherein the connection between the first and second components is a DC electric conductor segment that conducts the DC current from the first component to the second component and then from the second component back to the first component, the second component including manual controls for producing controlled manipulation of the DC current to thereby modify the temperature setting for the blanket.

3. A patient heating system as defined in claim 2 wherein the second component of said control comprises push-buttons that enable a caregiver to readily, manually and conveniently provide the temperature setting.

4. A patient heating system as defined in claim 3 wherein said push buttons are independently configured to allow distinction by feel as to the button for decreasing or increasing the temperature setting.

5. A patient heating system comprising:
a covering responsive to electrical current input from an electrical power source to provide variable warming of a patient;
a dual component controller including a first component for receiving electrical current from a power source and a second component structurally separate from and electrically connected to said first component to provide limited independent physical movement of said second component relative to said covering and first component;
a conductor for conducting electricity from said first component to said covering, and said second component comprising a hand held control enabling manipulation of the electrical current as conducted by said first component to the covering and thereby the heat temperature setting for the covering.

6. A patient heating system as defined in claim 5 wherein the second component is connected to the first component by a separate elongate flexible wire conductor.

7. A patient heating system as defined in claim 5 wherein the second component is configured for convenient manipulation by a caregiver and including securement means for securement of the second component on his person or surrounding for convenient manipulation by the caregiver.

8. A patient heating system as defined in claim 7 wherein the second component includes temperature up and down push-button control of the temperature.

9. A patient heating system as defined in claim 8 wherein the push-button controls are configured to enable a caregiver to readily distinguish by tactile feel as between temperature up control and temperature down control.

10. A patient heating system as defined in claim 6 wherein the electrical connection between the components is provided by a secondary wire conductor.

11. A patient heating system as defined in claim 5 wherein the electrical connection between the components is provided by cordless remote control.

12. A heating system for an animal being treated by a veterinarian including;
an operating table for accommodating an animal to be treated;
a covering for the animal comprised of electrifiable fabric that is responsive to electrical input for increasing and decreasing the heat generated by the covering;
a source of electricity and a multi-segment conductor and control for conducting electricity from the source to the covering and defining a path of conduction;
a first segment conducting said electricity from the source to a dual component controller including a first component that receives and then transmits electrical current to the covering;
a second component electrically connected to the first component and independently located out of the path of conduction for convenient location relative to the veterinarian, said second component including controls for controlling the degree of electrical power being conveyed to the covering.

13. A system for generating heat to a covering comprising:
a power source;
a conductor segment conducting electricity from said power source to a breakout member whereat dual conductor segments, including conductive wires that are bundled in the conductor segments, extend independently to a control member and to an electrifiable warming covering;
said electricity as extended to said covering being manipulable to desirably increase and decrease the temperature of said covering;
said control member including a pulse width modulator that enables the setting of a ratio that is readable by the control member whereby increasing and decreasing of the covering temperature is enabled with minimum wires in the conductor segments, and is insensitive to supply voltage variances from the nominal voltage.
